# FASCICULE DE BREVET EUROPEEN

(11) **EP 1 404 584 B1**
(45) Date de publication et mention de la délivrance du brevet: **08.12.2004**
(21) Numéro de dépôt: 02764944.1
(22) Date de dépôt: 05.07.2002
(51) Int. Cl.: B65D 41/20, B65D 81/32, A61M 5/28

(54) **PAROIS DE SEPARATION/FERMETURE D'UN CONTENANT**
WAND ZUM TRENNEN/VERSCHLIESSEN EINES BEHÄLTERS
SEPARATION AND/OR CLOSURE WALL FOR A CONTAINER AND METHODS FOR ASSEMBLING CONTAINERS USING SUCH A WALL

(30) Priorité: 05.07.2001 FR 0108913
(43) Date de publication de la demande: 07.04.2004
(73) Titulaire: INTERPHARM DEVELOPMENT, 1020 Remens (CH); Brevet, Michel, 12210 Laguiole (FR)
(72) Inventeur: PARIS, Laurence, F-03600 Commentry (FR); BRAS, Michel, F-12210 Laguiole (FR)
(74) Mandataire: Richebourg, Michel François
(86) Numéro de dépôt international: PCT/FR2002/002360
(87) Numéro de publication internationale: WO 2003/004368

(56) Documents cités:
- EP-A- 0 461 693
- GB-A- 1 192 817
- US-A- 3 695 478

## Description

### DOMAINE D'APPLICATION DE L'INVENTION

La présente invention a trait aux adaptations permettant d'assurer la fermeture d'un conditionnement, c'est à dire l'emballage dans un volume fermé d'au moins un produit à des fins de conservation avant son utilisation, puis l'ouverture du même conditionnement au moment de l'utilisation dudit produit dans des conditions optimales.

L'objet de la présente invention concerne également le double conditionnement c'est à dire l'emballage dans deux volumes différents de deux produits destinés à se retrouver en contact à des fins de mélange au moment de l'utilisation.

### DESCRIPTION DE L'ART ANTÉRIEUR

Le développement du marché de l'alimentaire, de la chimie, de la pharmaceutique, de la diététique et de la cosmétique a amené la création de nouveaux produits constitués de plusieurs substances qui ne doivent être mélangées qu'au moment de leur utilisation.

La séparation entre les deux substances à mélanger doit être nette pour respecter la conservation des produits avant leur utilisation notamment lorsque que le mélange de ceux-ci n'est possible que peu de temps avant leur emploi en raison de la réaction physico-chimique provoquée par le mélange, comme c'est souvent le cas dans le domaine pharmaceutique pour les produits injectables et produits pâteux tels que les crèmes et les gels ou dans le domaine de la fabrication de certains durcisseurs de colles rapides ou de la création des couleurs en peinture où les volumes des deux peintures doivent être chacun dosés de façon à fournir après mélange, une teinte particulière résultant du mélange entre les deux peintures.

Dans le domaine de l'alimentaire, la séparation des substances devient importante, et ce pour plusieurs raisons, parmi lesquelles les principales sont :
- l'esthétisme, car l'oeil du client dans un rayon sera plus attiré par un produit bi ou multi couleurs,
- la nouveauté, car la présence de deux substances dans un même paquet ne fait qu'apparaître,
- le goût, car le fait de réaliser le mélange au dernier moment garantit la distinction des saveurs ainsi que la préservation des goûts,
- la possibilité pour le client de choisir de mélanger ou pas, c'est à dire de commencer par une substance et de finir par l'autre.

La plupart des produits multi-couches alimentaires aujourd'hui présents sur le marché, utilise pour éviter le mélange avant consommation, un concept jouant sur la différence de viscosité et de masse volumique existante entre les produits constituant les couches afin qu'ils ne se mélangent pas entre eux. Cette solution bien que très utilisée ne garantit pas le non mélange notamment lors du transport qui peut, lors de chocs mal amortis, provoquer le mélange instantané et involontaire des deux substances, le produit devenant ainsi difficilement commercialisable.

Une solution à ces problèmes de migration et de mélange des substances constituant le produit à vendre réside dans un nouveau concept de conditionnement à savoir le double conditionnement.

Un dispositif de double conditionnement est décrit dans les brevets français n° 2633250 et 2783804 proposant un conditionnement réalisant un bloc homogène et étanche superposant deux compartiments, un des récipients présentant un orifice operculé sur sa surface extérieure perçable par un moyen extérieur dont est muni ledit conditionnement, ledit moyen de perçage servant également à percer la séparation des deux compartiments afin que puissent se mélanger les deux contenus. Un inconvénient à ce dispositif est que le perçage de l'opercule de séparation n'assure pas toujours la communication efficace entre les deux compartiments superposés de sorte que le mélange n'est pas assuré dans de bonnes conditions.

Le double conditionnement tel qu'il existe actuellement peut également se présenter sous d'autres formes, telles que :
- un sachet collé au récipient principal,
- un paquet emboîté au paquet principal,
- un produit à ajouter logé à l'intérieur du couvercle.

Les demanderesses ont constaté que ce nouveau concept de double conditionnement, bien que résolvant les problèmes de migration entre les produits, enlève l'unité du produit c'est à dire que le sachet ou le paquet "bis" est considéré comme accessoire par le consommateur. En effet, le consommateur délaisse souvent le conditionnement "bis" et ceci, pour plusieurs raisons :
- par négligence,
- du fait qu'il soit obligé de réaliser une ouverture supplémentaire sur un paquet auxiliaire,
- du fait qu'il ne perçoit pas l'utilité de celui-ci et,
- du fait du manque d'unité, qu'il ne perçoit pas sa complémentarité immédiate avec le premier conditionnement.

Il existe dans l'art antérieur plusieurs dispositifs permettant d'obvier aux inconvénients précités. Ainsi, par exemple, le dispositif, décrit et représenté dans le texte du brevet français n° 2507573, consiste en un récipient bi-compartimenté dont la paroi de séparation des deux compartiments est perforable par un dispositif de perçage disposé à l'intérieur du récipient. Ce dispositif a pour désavantage d'être manoeuvrable seulement au moyen d'une paille qui assure le mouvement du dispositif de perçage vers la paroi de séparation des deux compartiments. De plus, les conditions d'assemblage ne sont pas évidentes. En effet, selon le mode de réalisation illustré, les deux compartiments seraient operculés séparément puis assemblés de façon à ce que leur paroi de séparation se superpose et que le dispositif de perçage assure le déchirement des deux.

La difficulté dans les double conditionnements réside bien dans la destruction de la paroi de séparation et/ou de fermeture des deux conditionnements et dans le mode d'assemblage de ces deux derniers . En effet, bien que le concept en lui-même permette le remplissage séparé des contenants puis leur assemblage, plusieurs problèmes se posent lors de la mise en oeuvre au niveau de l'hygiène et notamment du maintien des conditions d'hygiène jusqu'à l'assemblage final.

De plus, un double conditionnement demande théoriquement deux fois plus d'opérations d'assemblage et deux fois plus de matières d'oeuvre qui grèvent considérablement le coût des produits présentés dans de tels emballages. Ainsi par exemple, le dispositif décrit par le susdit brevet superpose deux opercules ou équivalents.

Un autre dispositif de l'art antérieur décrit et représenté dans le brevet européen n° 0232814 procède d'un récipient bi-compartimenté dont le couvercle comporte un dispositif de perçage. La déformation du récipient dans sa totalité permet le perçage de la paroi de séparation et le mélange des deux substances stockées séparément. L'ouverture du récipient se fait par le haut et n'enlève pas toujours la paroi de séparation déchirée du récipient. De plus, le remplissage et l'obturation par les opercules dans de bonnes conditions et notamment dans des conditions alimentaires acceptables semblent difficiles à mettre en oeuvre dans les modes de réalisation représentés.

Un autre dispositif décrit et représenté dans le brevet européen n° 0173547 propose un récipient composé de trois parties, à savoir :
- un premier contenant principal rempli d'une première substance,
- un deuxième contenant se logeant, tout en le fermant, dans le premier compartiment en prenant appui sur le rebord de son ouverture , et
- un couvercle assurant la fermeture du deuxième contenant et le perçage du fond du deuxième compartiment.

Ce concept de bi-compartimentation a pour inconvénient le perçage du fond d'un compartiment et non d'un opercule ce qui demande un effort supplémentaire pour l'utilisateur ainsi que des matériaux beaucoup plus onéreux pour le dispositif de perçage et pour les liaisons entre le premier et le deuxième contenants.

Un autre dispositif décrit et représenté dans le brevet américain n° 3,695,478 et dans le brevet européen n°0461693 propose un bouchon élastiquement déformable pour seringue à injection qui a pour objet de séparer l'ampoule de l'aiguille d'injection de la seringue. La membrane formant la partie supérieure du bouchon éclate sous l'effet du perçage de l'aiguille et se retracte radialement vers la bride du bouchon pour former une ouverture permanente.

Un autre dispositif décrit et représenté dans le brevet britannique n° 1,192,817 propose un conditionnement pour amagalme dentaire prédosé qui comporte deux compartiments renfermant deux substances qui, destinées à être mélangées qu'au moment de l'emploi, sont séparées par une membrane intégrant une zone de fragilisation ou de rupture susceptible d'être déchirée par un moyen de déchirure telle qu'une masselotte interne entraînée par le coulissement axial d'un compartiment dans l'autre. Ce principe qui n'assure qu'un déchirement de la membrane , impose en outre la présence dans un compartiment d'un corps étranger (masselotte) qui devra bien entendu être enlevé du mélange final.

### DESCRIPTION DE L'INVENTION

Partant de cet état de fait, les demanderesses ont donc mené des recherches visant à améliorer ce nouveau concept de double conditionnement, recherches qui ont abouti à la conception d'une nouvelle paroi de séparation et/ou de fermeture d'un contenant permettant d'obvier aux inconvénients précités des doubles conditionnements existants tout en assurant le stockage séparé des substances jusqu'à leur mélange avant emploi dans des conditions optimales et sans risque de migration entre les deux substances.

Le problème que proposent de résoudre les demanderesses pour atteindre un tel objectif, repose en fait sur une paroi de séparation et/ou de fermeture qui soit capable dans un premier temps, de couvrir l'ouverture d'au moins un contenant pour retenir une substance admise à l'intérieur à des fins de conservation avant utilisation, et, dans un deuxième temps, d'être déchirée pour découvrir ladite ouverture et libérer ainsi par cette dernière, la substance hors de la cavité dudit contenant à des fins d'utilisation.

Pour y parvenir, elles ont imaginé une paroi de séparation et/ou de fermeture d'un contenant reposant sur une membrane déformable disposée sur le pourtour de l'ouverture dudit contenant pour couvrir cette dernière avec une tension lui permettant d'éclater sous l'effet d'un perçage et/ou d'un déchirement, pour se rétracter sur le pourtour de ladite ouverture et découvrir entièrement la susdite ouverture. La présence d'une membrane déformable tendue sur le pourtour de l'ouverture d'un contenant offre de grands avantages tant sur la fonction de fermeture que sur celle d'ouverture du contenant. En effet, de par sa grande souplesse à la déformation, la membrane assurera au contenant fermé, une meilleure résistance aux chocs, et de par sa faculté à éclater sous l'effet d'un simple perçage ou d'un simple pincement pour se rétracter totalement sur le pourtour de l'ouverture du contenant, la même membrane assurera une découverte complète de ladite ouverture. Il va de soi que l'homme de métier mettra en adéquation tous les paramètres physico-chimiques du matériau utilisé, pour la membrane déformable afin de déterminer la compatibilité avec la substance retenue dans la cavité du contenant qu'elle ferme et, par une étude dynamométrique classique, l'aptitude à la flexion et/ou à la résistance de la membrane tendue sous l'effet de chocs d'objets contondants et/ou de la poussée de la substance retenue à l'intérieur du contenant avec son aptitude au déchirement immédiat et à l'éclatement sous l'effet d'une poussée d'un objet perçant ou coupant ou d'un pincement par torsion de la dite membrane. Les propriétés physiques de cette membrane déformable rappellent ainsi celles de la membrane d'un ballon qui, lorsqu'il est gonflé d'un fluide gazeux augmente de volume et accepte une flexion quand on le comprime avec les mains (objet contondant) alors qu'il éclate quand il rencontre une épine de rosier (objet perçant ou coupant). Un autre avantage d'une telle membrane déformable qui, pour une production en série industrielle, pourra, selon un procédé connu de fabrication, se présenter sous la forme d'un film étiré qui sera ajusté et maintenu sur le pourtour des ouvertures de contenants juxtaposés, est d'augmenter la surface du film pour couvrir lesdites ouvertures, réduisant amplement le prix de revient des parois de fermeture ainsi réalisées. Un autre avantage d'une telle membrane déformable est son adaptabilité à tout profil d'ouverture et donc à tout contenant actuellement sur le marché.

Il va de soi que par rapport aux autres dispositifs décrits de l'art antérieur, le moyen d'ouverture du pourtour du contenant n'est assuré ici que par la faculté à l'éclatement de la membrane qui assumait auparavant la fermeture du contenant, éclatement qui ne pouvait être autorisé que par une mise sous tension préalable de la même membrane. Il ne s'agit donc pas ici d'une tension pour maintenir un orifice constant le temps du passage d'une aiguille et pour se refermer ensuite ou encore d'une zone de fragilisation d'une membrane susceptible d'être déchirée par une masselotte.

Selon une caractéristique particulièrement avantageuse de l'invention, la susdite membrane déformable de la paroi de séparation et/ou de fermeture est façonnée dans un matériau souple afin d'accepter plus facilement les déformations à la tension, et notamment à la flexion lorsqu'elle est tendue pour résister aux chocs intempestifs auxquels sont généralement soumis les contenants.

Selon une autre caractéristique particulièrement avantageuse de l'invention, la susdite membrane déformable de la paroi de séparation et/ou de fermeture est façonnée dans un matériau élastique afin d'accepter une plus grande déformation à la tension et d'offrir une meilleure rétractabilité sur le pourtour de l'ouverture sous l'effet d'un déchirement, selon le module d'élasticité du matériau utilisé. Comme indiqué plus haut, ce module d'élasticité sera également en adéquation avec les autres paramètres physico-chimiques du matériau instruit judicieusement pour permettre à la membrane tendue sur le pourtour de l'ouverture de résister sans se déchirer sous l'effet de chocs d'objets contondants et/ou de la poussée de la substance retenue à l'intérieur du contenant tout en se déchirant et en éclatant avec peu de flexion sous l'effet d'un contact avec un objet coupant ou perçant ou sous l'effet d'une torsion entraînant un pincement suivi d'un déchirement de la dite membrane ou par combinaison des deux modes d'éclatement de la dite membrane.

Selon une autre caractéristique particulièrement avantageuse de l'invention, la susdite membrane déformable de la paroi de séparation et/ou de fermeture est façonnée dans un matériau plastique qui facilitera la mise en place de la membrane sur le pourtour de l'ouverture du contenant en acceptant une déformation à la tension et un scellage hermétique sur le pourtour de l'ouverture par sertissage, par collage, ou par tout autre technique équivalente connue de l'homme de métier.

Selon une autre caractéristique particulièrement avantageuse de l'invention, la susdite membrane déformable de la paroi de séparation et/ou de fermeture est disposée de manière étanche sur le pourtour de l'ouverture du contenant afin d'assurer une séparation absolue entre l'intérieur et l'extérieur du contenant fermé par ladite membrane. Cette disposition autorise le remplissage sous vide du contenant.

Selon un premier mode de réalisation de l'invention, la susdite membrane déformable de la paroi de séparation et/ou de fermeture est tendue directement sur le pourtour de l'ouverture dudit contenant. Ce premier mode de réalisation sera particulièrement adapté lorsque la chaîne de production assurant la mise en place de la membrane disposera des contenants. Ce cas de figure qui, sera certes le plus fréquemment employé pour des raisons de rentabilité, pourra être mis en oeuvre aussi bien sur des contenants remplis de la substance que sur des contenants vides dont le remplissage dans une unité de remplissage distincte de l'unité de pose de la membrane sera assuré ultérieurement par une autre ouverture qui sera fermée classiquement par un couvercle ou un opercule scellable sur son pourtour.

Selon un deuxième mode de réalisation de l'invention, la susdite membrane déformable de la paroi de séparation et/ou de fermeture est tendue sur un support rapporté sur le pourtour de l'ouverture dudit contenant. Ce deuxième mode de réalisation sera, quant à lui, particulièrement adapté lorsque la chaîne de production assurant la mise en place de la membrane ne disposera pas des contenants, c'est à dire lorsque le support avec sa membrane tendue sera installé sur le pourtour de l'ouverture du contenant par une unité de production par exemple celle qui assurera le remplissage de la substance dans la cavité du contenant.

Telle que décrite ci-dessus, la membrane de l'invention trouve une application directe pour faire office de paroi de séparation et/ou de fermeture d'un contenant considéré comme le conditionnement simple et classique d'un produit en vue de sa présentation à la vente tels que les conditionnements plus communément connus sous l'appellation de conditionnements dits "blister", c'est à dire une coque en plastique transparent collée sur un support déchirable et sous laquelle sont vendues certaines marchandises, comme les comprimés, les gélules ou les produits lyophilisés pharmaceutiques ou non et présentés sous forme de plaquettes avec plusieurs alvéoles dans lesquelles lesdits produits pourront être retenus par la membrane de l'invention.

Cependant, l'application première du nouveau concept de la paroi de séparation et/ou de fermeture de la présente invention qui a suscité les principaux travaux de recherche des demanderesses est le double conditionnement, c'est à dire l'emballage dans des volumes différents de plusieurs substances destinées à se retrouver en contact à des fins de mélange au moment de leur utilisation. Pour répondre à ce besoin, les demanderesses ont ainsi improvisé, selon le concept fondamental de l'invention, de disposer une membrane déformable, tendue entre au moins deux volumes de substances afin de former dans un premier temps, une barrière physique entre les volumes desdites deux substances délimités par ladite membrane dans au moins un contenant à des fins de conservation isolée des substances et, dans un deuxième temps, par déchirement et éclatement de la membrane tendue, un moyen de mise en communication desdits volumes afin d'assurer par un bon écoulement des substances entre les deux volumes, le mélange desdites deux substances à des fins d'utilisation de ce dernier.

L'utilisation d'une paroi de séparation et/ou de fermeture à partir de l'utilisation d'une membrane déformable installée sous tension et qui éclate sous l'effet d'un contact avec un objet perçant ou coupant ou par déchirement sous l'effet d'une torsion ou la combinaison des deux modes d'éclatement de la dite membrane, permet d'améliorer considérablement le concept de double conditionnement sus-évoqué avec d'une part, les deux volumes séparés physiquement avant utilisation, pour que le double conditionnement soit respecté en évitant tout problème de migration entre les deux substances et d'autre part, les deux volumes en libre communication après éclatement de la membrane, pour établir correctement le mélange. Avantageusement, associée de manière étanche sur un des contenants cette membrane autorise le remplissage sous vide de ce contenant et ainsi une aspiration de l'autre substance ce qui facilitera grandement l'opération de mélange des deux substances. En outre, les techniques d'assemblage ou de création des compartiments pourront être adaptées à n'importe quelle unité de production, que l'opération de pose des membranes soit postérieure ou antérieure à l'opération de remplissage des contenants ou que les deux unités de pose et de remplissage des contenants soient distinctes ou non.

Ainsi, un premier mode d'assemblage de deux contenants consiste :
- à tendre une membrane déformable selon l'invention sur le pourtour de l'ouverture d'un premier contenant dans une unité de pose,
- à remplir le premier contenant par une première substance et à fermer ledit premier contenant par exemple au moyen d'un opercule scellable, dans une unité de remplissage qui peut être éloignée de l'unité de pose,
- à remplir le deuxième contenant par une deuxième substance via une ouverture de remplissage, dans une unité de remplissage qui peut être la même ou différente de l'unité de remplissage du premier contenant,
- et à assembler les deux contenants par scellage, clippage ou vissage du premier contenant sur le deuxième contenant en liant entre eux les pourtours des ouvertures des deux contenants de telle sorte que les deux contenants soient séparés avec leur ouverture en regard l'une de l'autre, via la paroi de séparation que forme la susdite membrane de fermeture.

Ce premier mode d'assemblage qui s'opère par superposition des deux contenants réunis par leurs ouvertures et dont les volumes sont cloisonnés par la membrane tendue, permet entre autres de pouvoir différencier l'unité de pose de la membrane de l'unité de remplissage des contenants d'autant que bien souvent la première unité relève de chaînes de production spécialisée en conditionnement et la deuxième unité relève de chaînes de production spécialisée en approvisionnement des substances. En outre, si les substances utilisées devaient être produites dans des unités distinctes, ce premier mode d'assemblage trouve une application particulièrement dédiée dans la mesure où il peut autoriser la fabrication des contenants dans une unité, la pose de la membrane déformable dans une autre unité, le remplissage du premier contenant dans une autre unité et le remplissage du second contenant encore dans une autre unité. Ainsi par exemple dans le domaine alimentaire, un des contenants peut être sous-traité donnant ainsi la possibilité à une entreprise spécialisée dans les produits laitiers de proposer des produits avec double conditionnement sans que celle-ci ne diversifie sa production en sous-traitant l'un des contenants à une entreprise spécialisée dans la fabrication du produit à ajouter, distinct du produit laitier.

Un deuxième mode d'assemblage de deux contenants consiste :
- à remplir le premier contenant par une première substance via une ouverture de remplissage, dans une première unité de remplissage
- à fermer la susdite ouverture de remplissage du premier contenant en tendant la susdite membrane de la paroi de séparation et/ou de fermeture sur le pourtour de l'ouverture de remplissage, dans une unité de pose qui pourra être intégrée à la première unité de remplissage,
- à remplir le deuxième contenant par une deuxième substance via une ouverture de remplissage, dans une unité de remplissage qui peut être la même ou différente de la première unité de remplissage,
- et à assembler les deux contenants par scellage, clippage ou vissage du premier contenant sur le deuxième contenant en liant entre eux les pourtours des deux ouvertures de remplissage des deux contenants de telle sorte que les deux contenants soient séparés avec leur ouverture en regard l'une de l'autre, via la paroi de séparation que forme la susdite membrane de fermeture.

Ce deuxième mode d'assemblage permet de maîtriser dans une même chaîne de production, l'unité de pose de la membrane déformable et les unités de remplissage des contenants.

Un troisième mode d'assemblage d'assemblage de deux contenants consiste :
- à tendre la susdite membrane déformable de la paroi de séparation et/ou de fermeture sur le pourtour de l'ouverture d'un premier contenant, dans une unité de pose,
- à remplir le deuxième contenant par une deuxième substance via une ouverture d'admission, dans une première unité de remplissage qui peut être différente de l'unité de pose,
- à assembler les deux contenants par engagement hermétique du premier contenant dans l'ouverture d'admission du deuxième contenant en prédisposant la membrane à l'intérieur du deuxième contenant de telle sorte que les deux contenants ne soient séparés que par la susdite membrane de fermeture,
- à remplir le premier contenant par une première substance, dans une deuxième unité de remplissage qui peut être la même ou différente de la première unité de remplissage,
- et à fermer simultanément les ouvertures deux contenants.

Ce troisième mode d'assemblage offre les mêmes possibilités que celles du premier mode d'assemblage par rapport aux unités de pose et de remplissage. Cependant, comme celui qui a été rappelé ci-dessus dans le brevet européen n° 0173547, il propose d'assembler différemment les contenants en prévoyant de loger le premier dans le deuxième tout en le fermant et en prenant appui sur le rebord de son ouverture, avec par exemple un couvercle assurant concomitamment la fermeture hermétique des deux contenants. Ce mode d'assemblage évite ainsi un assemblage par scellage des deux contenants au niveau de la membrane.

Un quatrième mode d'assemblage de deux contenants consiste :
- à remplir le premier contenant par une première substance via une ouverture de remplissage, dans une première unité de remplissage
- à fermer la susdite ouverture de remplissage du premier contenant en tendant la susdite membrane déformable de la paroi de séparation et/ou de fermeture sur le pourtour de l'ouverture de remplissage, dans une unité de pose qui pourra être intégrée à la première unité de remplissage,,
- à remplir le deuxième contenant par une deuxième substance via une ouverture de remplissage, dans une deuxième unité de remplissage qui peut être la même ou différente de la première unité de remplissage,
- à assembler les deux contenants par engagement hermétique du premier contenant dans l'ouverture de remplissage du deuxième contenant en prédisposant la membrane à l'intérieur du deuxième contenant de telle sorte que les deux contenants ne soient séparés que par la susdite membrane de fermeture.

Ce quatrième mode d'assemblage offre les mêmes possibilités que celles du deuxième mode d'assemblage par rapport aux unités de pose et de remplissage et les mêmes possibilités que celles du troisième mode d'assemblage par rapport à la réunion proprement dite des deux contenants.

Un cinquième mode d'assemblage concernant un seul contenant consiste :
- à tendre la susdite membrane déformable de la paroi de séparation et/ou de fermeture sur un support, dans une unité de pose,
- à remplir le premier compartiment du contenant par une première substance via l'ouverture de remplissage, dans une première unité de remplissage qui peut être différente de l'unité de pose,
- à positionner ledit support avec la membrane tendue, sur les contours d'un épaulement interne du contenant, via la susdite ouverture de remplissage,
- à remplir le deuxième compartiment situé de l'autre côté de la membrane du contenant par une deuxième substance via ladite ouverture de remplissage, dans une deuxième unité de remplissage qui peut être la même ou différente de la première unité de remplissage,
- et à fermer la susdite ouverture de remplissage du contenant.

Ce cinquième mode d'assemblage qui s'applique plus précisément à un contenant possédant un fond et une ouverture de remplissage, permet de cloisonner un même contenant en un ou plusieurs compartiments selon le nombre de supports installés avec des unités de pose et de remplissage qui pourront être les mêmes ou différentes.

Un sixième mode d'assemblage concernant un seul contenant consiste :
- à tendre la susdite membrane déformable de la paroi de séparation et/ou de fermeture sur un support, dans une unité de pose,
- à positionner ledit support avec la membrane tendue sur les contours d'un épaulement interne du contenant,
- à remplir le premier compartiment du contenant par une première substance via la première ouverture de remplissage, dans une première unité de remplissage qui peut être différente de l'unité de pose,
- à remplir le deuxième compartiment situé de l'autre côté de la membrane du contenant par une deuxième substance via la deuxième ouverture de remplissage, dans une deuxième unité de remplissage qui peut être la même ou différente de la première unité de remplissage,
- et à fermer les deux ouvertures de remplissage des deux compartiments du susdit contenant.

Ce sixième mode d'assemblage diffère du cinquième en ce qu'il s'applique à un contenant possédant deux ouvertures de remplissage opposées.

On comprend que la paroi de séparation et/ou de fermeture et les modes d'assemblage des contenants, qui viennent d'être ci-dessus décrits, l'ont été en vue d'une divulgation plutôt que d'une limitation et ont pour objet de mettre en exergue l'expression la plus élémentaire du concept fondamental de l'invention qui repose sur l'utilisation originale d'une membrane déformable tendue sur le pourtour d'une ouverture pour assurer la fermeture d'une cavité d'un contenant. C'est pourquoi le moyen d'éclatement de la membrane par un organe perçant ou coupant ou par torsion de cette dernière engendrant pincement, déclenché par une action manuelle de l'utilisateur/consommateur n'a pas été décrit dans le présent mémoire car sans implication directe avec le concept de l'invention. Le mode d'éclatement de la membrane pourra en effet être facilement mis en oeuvre selon le mode d'assemblage utilisé. Celui-ci pourra être obtenu à l'aide d'un objet de perçage comme ceux décrits dans les nombreux brevets de l'art antérieur tel celui logé à l'intérieur d'un des contenants, ou celui situé à l'extérieur ou entre les deux ou encore celui formé par la paroi d'un des contenants pour ne pas ajouter de dispositif supplémentaire à l'assemblage des deux contenants. Un autre mode d'éclatement de la membrane sera obtenu sans objet de perçage par simple augmentation de la tension de la membrane sous l'effet d'un clippage accentué du conditionnement supérieur sur le conditionnement inférieur ou sous l'effet d'un vissage du conditionnement supérieur dans le conditionnement inférieur. Dans ce cas il y a torsion de la membrane engendrant une augmentation de la tension de la membrane associé à un phénomène de pincement conduisant à un déchirement de cette dernière. Le phénomène de clippage ou de torsion peut être combiné à la présence d'un objet de perçage disposé à l'intérieur d'un des compartiments et solidaire de la paroi de ces derniers. De même, la membrane pourra être réalisée dans un matériau simple ou composite et/ou dans un matériau mono-lamellaire ou pluri-lamellaire et/ou avec incorporation ou non d'une substance isolante tel que de l'aluminium, du téflon ou similaire, et/ou avec ses deux faces traitées pour être compatibles avec les deux milieux avec lesquels elle est en contact. Cette même membrane pourra être sélective vis-à-vis des gaz en contact avec elle laissant passer ou non soit l'azote, soit l'oxygène soit le gaz carbonique permettant ainsi une meilleure conservation des produits contenus dans l'un et l'autre compartiment, en fonction du type de membrane retenue.

Comme dans le domaine alimentaire, le double conditionnement est aussi applicable dans le domaine pharmaceutique et notamment dans le cas des seringues à double compartiment dans laquelle une première substance est retenue dans le corps de la seringue et une deuxième substance est retenue dans celui du piston, ce dernier étant obturé par une membrane de l'invention disposée au contact de la première substance de sorte que ladite membrane fasse office de paroi de séparation entre les deux substances qui peuvent être aussi bien deux liquides qu'une poudre et un liquide. La mise en contact des deux substances est obtenue par simple pression sur le piston entraînant ainsi l'éclatement de la membrane par l'intermédiaire d'un obstacle perçant placé à l'intérieur du corps de la seringue solidaire ou non de ce dernier, ou par vissage du piston dans le corps de la seringue engendrant une torsion de la membrane entraînant sa rupture.

## Revendications

1. Paroi de séparation d'un conditionnement en au moins deux compartiments destinée dans un premier temps, à couvrir l'ouverture d'au moins un compartiment pour retenir une substance admise à l'intérieur à des fins de conservation avant utilisation, et dans un deuxième temps à être déchirée pour découvrir ladite ouverture et libérer la substance hors dudit compartiment dans le second à des fins d'utilisation, **CARACTÉRISÉE EN CE QUE** la susdite paroi de séparation est constituée par une membrane déformable qui, disposée sous tension sur le pourtour de l'ouverture dudit compartiment pour couvrir cette dernière, est susceptible, par éclatement sous l'effet d'un perçage ou d'un déchirement, de se rétracter sur le pourtour de ladite ouverture et découvrir entièrement la susdite ouverture, comme la membrane d'un ballon qui, lorsqu'il est gonflé d'un fluide gazeux augmente de volume et accepte une flexion quand on le comprime avec les mains alors qu'il éclate quand il rencontre une épine de rosier.

2. Paroi de séparation selon la revendication 1, **CARACTÉRISÉE EN CE QUE** la susdite membrane déformable de la paroi de séparation est disposée de manière étanche sur le pourtour de l'ouverture d'un desdits compartiments.

3. Paroi de séparation selon la revendication 1, **CARACTÉRISÉE EN CE QUE** la susdite membrane déformable de la paroi de séparation est façonnée dans un matériau souple.

4. Paroi de séparation selon la revendication 1, **CARACTÉRISÉE EN CE QUE** la susdite membrane déformable de la paroi de séparation est façonnée dans un matériau élastique et/ou plastique.

5. Paroi de séparation selon la revendication 1, **CARACTÉRISÉE EN CE QUE** la susdite membrane déformable de la paroi de séparation est façonnée dans un matériau simple.

6. Paroi de séparation selon la revendication 1, **CARACTÉRISÉE EN CE QUE** la susdite membrane déformable de la paroi de séparation est façonnée dans un matériau composite.

7. Paroi de séparation selon la revendication 1, **CARACTÉRISÉE EN CE QUE** la susdite membrane déformable de la paroi de séparation est façonnée dans un matériau mono ou pluri-lamellaire.

8. Paroi de séparation selon la revendication 1, **CARACTÉRISÉE EN CE QUE** la susdite membrane déformable de la paroi de séparation est façonnée dans un matériau contenant une substance isolante.

9. Paroi de séparation selon la revendication 1, **CARACTÉRISÉE EN CE QUE** la susdite membrane déformable de la paroi de séparation est façonnée dans un matériau présentant une sélectivité aux gaz.

10. Paroi de séparation selon la revendication 1. **CARACTÉRISÉE EN CE QUE** la susdite membrane déformable de la paroi de séparation est disposée directement sur le pourtour de l'ouverture d'un desdits compartiments.

11. Paroi de séparation selon la revendication 1, **CARACTÉRISÉE EN CE QUE** la susdite membrane déformable de la paroi de séparation est tendue sur un support rapporté sur le pourtour de l'ouverture d'un desdits compartiments.

12. Paroi de séparation selon l'une quelconque des revendications 1 à 11, **CARACTÉRISÉE EN CE QUE** la membrane déformable de la susdite paroi de séparation est disposée sous tension entre au moins deux volumes de substances afin de former dans un premier temps, une barrière physique entre les volumes desdites deux substances délimités par ladite membrane dans au moins un compartiment à des fins de conservation isolée des substances et, dans un deuxième temps, par déchirement et éclatement de la membrane tendue par un objet perçant et/ou coupant ou pincement sous l'effet d'une torsion de cette dernière, un moyen de mise en communication desdits volumes afin d'assurer le mélange desdites deux substances à des fins d'utilisation de ce dernier.

13. Procédé d'assemblage d'au moins deux compartiments renfermant chacun une substance cloisonnée l'une de l'autre par une paroi de séparation selon la revendication 12, **CARACTÉRISÉ EN CE QU'**il consiste :
- à tendre la susdite membrane déformable de la paroi de séparation sur le pourtour de l'ouverture d'un premier compartiment,
- à remplir le premier compartiment par une première substance et à fermer ledit deuxième compartiment,
- à remplir le deuxième compartiment par une deuxième substance via une ouverture de remplissage,
- et à assembler les deux compartiments par scellage, clippage ou vissage du premier compartiment sur le deuxième compartiment en liant entre eux les pourtours des ouvertures des deux compartiments de telle sorte que les deux compartiments soient séparés avec leur ouverture en regard l'une de l'autre, via la paroi de fermeture que forme la susdite membrane de séparation.

14. Procédé d'assemblage d'au moins deux compartiments renfermant chacun une substance cloisonnée l'une de l'autre par une paroi de séparation selon la revendication 12, **CARACTéRISé EN CE QU'**il consiste :
- à remplir le premier compartiment par une première substance via une ouverture de remplissage.
- à fermer la susdite ouverture de remplissage du premier compartiment en tendant la susdite membrane déformable de la paroi de séparation sur le pourtour de l'ouverture de remplissage,
- à remplir le deuxième compartiment par une deuxième substance via une ouverture de remplissage,
- et à assembler les deux compartiments par scellage, clippage ou vissage du premier compartiment sur le deuxième compartiment en liant entre eux les pourtours des deux ouvertures de remplissage des deux compartiments de telle sorte que les deux compartiments soient séparés avec leur ouverture en regard l'une de l'autre, via la paroi de séparation que forme la susdite membrane de fermeture.

15. Procédé d'assemblage d'au moins deux compartiments renfermant chacun une substance cloisonnée l'une de l'autre par une paroi de séparation selon la revendication 12, **CARACTéRISé EN CE QU'**il consiste :
- à tendre la susdite membrane déformable de la paroi de séparation sur le pourtour de l'ouverture d'un premier compartiment,
- à remplir le deuxième compartiment par une deuxième substance via une ouverture de remplissage,
- à assembler les deux compartiments par engagement hermétique du premier compartiment dans l'ouverture d'admission du deuxième compartiment en prédisposant la membrane à l'intérieur du deuxième compartiment de telle sorte que les deux compartiments ne soient séparés que par la susdite membrane de fermeture
- à remplir le premier compartiment par une première substance,
- et à fermer les deux compartiments.

16. Procédé d'assemblage d'au moins deux compartiments renfermant chacun une substance cloisonnée l'une de l'autre par une paroi de séparation selon la revendication 12, **CARACTéRISé EN CE QU'**il consiste :
- à remplir le premier compartiment par une première substance via une ouverture de remplissage,
- à fermer la susdite ouverture de remplissage du premier compartiment en tendant la susdite membrane déformable de la paroi de séparation sur le pourtour de l'ouverture,
- à remplir le deuxième compartiment par une deuxième substance via une ouverture de remplissage,
- à assembler les deux compartiments par engagement hermétique du premier compartiment dans l'ouverture de remplissage du deuxième compartiment en prédisposant la membrane à l'intérieur du deuxième compartiment de telle sorte que les deux compartiments ne soient séparés que par la susdite membrane de fermeture.

17. Procédé d'assemblage d'un compartiment comportant une seule ouverture de remplissage d'au moins deux substances cloisonnées l'une de l'autre par une paroi de séparation selon la revendication 12, **CARACTéRISé EN CE Qu'**il consiste :
- à tendre la susdite membrane déformable de la paroi de séparation sur un support,
- à remplir le premier compartiment du compartiment par une première substance via l'ouverture de remplissage,
- à positionner ledit support avec la membrane tendue sur les contours d'un épaulement interne du compartiment, via la susdite ouverture de remplissage,
- à remplir le deuxième compartiment situé de l'autre côté de la membrane du compartiment par une deuxième substance via ladite ouverture de remplissage,
- et à fermer la susdite ouverture de remplissage du compartiment.

18. Procédé d'assemblage d'un compartiment comportant deux ouvertures opposées de remplissage d'au moins deux substances cloisonnées l'une de l'autre par une paroi de séparation selon la revendication 12, **CARACTéRISé EN CE QU'**il consiste :
- à tendre la susdite membrane déformable de la paroi de séparation sur un support,
- à positionner ledit support avec la membrane tendue sur les contours d'un épaulement interne du compartiment,
- à remplir le premier compartiment du compartiment par une première substance via la première ouverture de remplissage,
- à remplir le deuxième compartiment situé de l'autre côté de la membrane du compartiment par une deuxième substance via la deuxième ouverture de remplissage,
- et à fermer les deux ouvertures de remplissage des deux compartiments du susdit compartiment.

## Patentansprüche

1. Wand zum Trennen einer Verpackung in mindestens zwei Fächer, die in erster Linie bestimmt ist, die Öffnung mindestens eines Fach abzudecken, um zu Aufbewahrungszwecken vor dem Einsatz eine Substanz zurückzuhalten, die intern zugelassen ist und in zweiter Linie dafür bestimmt ist, zerrissen zu werden, um die besagte Öffnung Fach freizulegen und die Substanz aus dem besagten Fach in dem zweiten zu Einsatzzwecken freizusetzen, **dadurch gekennzeichnet, dass** die obengenannte Trennwand aus einer verformbaren Membran besteht, die, wenn sie unter Spannung auf dem Umfang der Öffnung des besagten Fachs angeordnet ist, um letztere abzudecken, durch Platzen aufgrund einer Durchbohrung oder durch Zerreißen in der Lage ist, sich auf dem Umfang der besagten Öffnung zusammenzuziehen und die besagte Öffnung vollkommen freizulegen und zwar so, wie die Membran eines Druckbehälters, dessen Volumen zunimmt, wenn er mit einem gasförmigen Flüssigkeit aufgeblasen wird und eine Biegung zulässt, wenn man ihn mit den Händen zusammendrückt, während er platzt, wenn er auf einen Rosendorn trifft.

2. Trennwand gemäß Anspruch 1, **dadurch gekennzeichnet, dass** die obengenannte verformbare Membran der Trennwand dicht auf dem Umfang der Öffnung eines der genannten Fächer angeordnet ist.

3. Trennwand gemäß Anspruch 1, **dadurch gekennzeichnet, dass** die obengenannte verformbare Membran der Trennwand aus einem elastischen Werkstoff geformt ist.

4. Trennwand gemäß Anspruch 1, **dadurch gekennzeichnet, dass** die obengenannte verformbare Membran der Trennwand aus einem elastischen Werkstoff und/oder Kunststoff geformt ist.

5. Trennwand gemäß Anspruch 1, **dadurch gekennzeichnet, dass** die obengenannte verformbare Membran der Trennwand aus einem einfachen Werkstoff geformt ist.

6. Trennwand gemäß Anspruch 1, **dadurch gekennzeichnet, dass** die obengenannte verformbare Membran der Trennwand aus einem Verbundwerkstoff geformt ist.

7. Trennwand gemäß Anspruch 1, **dadurch gekennzeichnet, dass** die obengenannte verformbare Membran der Trennwand aus einem Werkstoff mit einer oder mehreren Lamellen geformt ist.

8. Trennwand gemäß Anspruch 1, **dadurch gekennzeichnet, dass** die obengenannte verformbare Membran der Trennwand aus einem Werkstoff geformt ist, der einen Isolierstoff enthält.

9. Trennwand gemäß Anspruch 1, **dadurch gekennzeichnet, dass** die obengenannte verformbare Membran der Trennwand aus einem Werkstoff geformt ist, der eine selektive Wirkung bei Gaseinfluss aufweist.

10. Trennwand gemäß Anspruch 1, **dadurch gekennzeichnet, dass** die obengenannte verformbare Membran der Trennwand direkt auf dem Umfang der Öffnung eines der genannten Fächer angeordnet ist.

11. Trennwand gemäß Anspruch 1, **dadurch gekennzeichnet, dass** die obengenannte verformbare Membran der Trennwand auf einem Träger gespannt ist, der auf dem Umfang der Öffnung eines der genannten Fächer angeordnet ist.

12. Trennwand gemäß einem der Ansprüche 1 bis 11, **dadurch gekennzeichnet, dass** die verformbare Membran der obengenannten Trennwand unter Spannung zwischen mindestens zwei Substanzvolumen angeordnet ist, um in erster Linie eine physikalische Sperre zwischen den Volumen der beiden genannten Substanzen zu bilden, die von der genannten Membran in mindestens einem Fach abgegrenzt werden, zum Zwecke einer isolierten Aufbewahrung der Substanzen und in zweiter Linie durch Zerreißen und Platzen der gespannten Membran durch einen Gegenstand, der die Membran durchbohrt und/oder zerschneidet oder durch Abschneiden durch die Einwirkung einer Drehung letzterer, ein Mittel zur Herstellung der Kommunikation der genannten Volumen, um die Mischung der beiden genannten Substanzen zum Einsatzwecke letzterer zu garantieren.

13. Verfahren zum Zusammenbau mindestens zweier Fächer, wobei jedes Fach eine Substanz enthält, die durch eine Trennwand von der anderen abgetrennt ist, gemäß Anspruch 12, **dadurch gekennzeichnet, dass** es Folgendes umfasst:
- Spannen der obengenannten verformbaren Membran der Trennwand auf dem Umfang der Öffnung eines ersten Fachs,
- Füllen des ersten Fachs mit einer ersten Substanz und Schließen des besagten zweiten Fachs,
- Füllen des zweiten Fachs mit einer zweiten Substanz über eine Füllöffnung,
- und Zusammenbau der beiden Fächer durch hermetisches Verschließen, mit Hilfe von Clips oder Verschrauben des ersten Fachs auf dem zweiten Fach, wobei die Umfänge der Öffnungen der beiden Fächer so verbunden werden, dass die beiden Fächer mit ihrer einander gegenüberliegenden Öffnung über die Absperrwand, die die obengenannte Trennmembran bildet, getrennt sind.

14. Verfahren zum Zusammenbau mindestens zweier Fächer, wobei jedes Fach eine Substanz enthält, die durch eine Trennwand von der anderen abgetrennt ist, gemäß Anspruch 12, **dadurch gekennzeichnet, dass** es Folgendes umfasst:
- Füllen des ersten Fachs durch eine erste Substanz über eine Füllöffnung,
- Schließen der obengenannten Füllöffnung des ersten Fachs, wobei die obengenannte verformbare Membran der Trennwand auf dem Umfang der Füllöffnung gespannt wird.
- Füllen des zweiten Fachs mit einer zweiten Substanz über eine Füllöffnung,
- und Zusammenbau der beiden Fächer durch hermetisches Verschließen, mit Hilfe von Clips oder Verschrauben des ersten Fachs auf dem zweiten Fach, wobei die Umfänge der beiden Füllöffnungen der beiden Fächer so verbunden werden, dass die beiden Fächer mit ihrer einander gegenüberliegenden Öffnung durch die Trennwand, die die obengenannte Absperrmembran bildet, getrennt sind.

15. Verfahren zum Zusammenbau mindestens zweier Fächer, wobei jedes Fach eine Substanz enthält, die durch eine Trennwand von der anderen abgetrennt ist, gemäß Anspruch 12, **dadurch gekennzeichnet, dass** es Folgendes umfasst:
- Spannen der obengenannten verformbaren Membran der Trennwand auf dem Umfang der Öffnung eines ersten Fachs,
- Füllen des zweiten Fachs mit einer zweiten Substanz über eine Füllöffnung,
- Zusammenbau der beiden Fächer durch hermetisches Einsetzen des ersten Fachs in die Einlassöffnung des zweiten Fachs, indem die Membran vorher im zweiten Fach so angeordnet wird, dass die beiden Fächer nur durch die obengenannte Absperrmembran getrennt sind,
- Füllen des ersten Fachs mit einer ersten Substanz,
- und Schließe der beiden Fächer.

16. Verfahren zum Zusammenbau mindestens zweier Fächer, wobei jedes Fach eine Substanz enthält, die durch eine Trennwand von der anderen abgetrennt ist, gemäß Anspruch 12, **dadurch gekennzeichnet, dass** es Folgendes umfasst:
- Füllen des ersten Fachs mit einer ersten Substanz über eine Füllöffnung,
- Schließen der obengenannten Füllöffnung des ersten Fachs durch Spannen der obengenannten verformbaren Membran der Trennwand auf dem Umfang der Öffnung,
- Füllen des zweiten Fachs mit einer zweiten Substanz über eine Füllöffnung,
- Zusammenbau der beiden Fächer durch hermetisches Einsetzen des ersten Fachs in die Füllöffnung des zweiten Fachs, indem die Membran vorher im zweiten Fach so angeordnet wird, dass die beiden Fächer nur durch die obengenannte Absperrmembran getrennt sind.

17. Verfahren zum Zusammenbau eines Fachs, das eine einzige Füllöffnung mit mindestens zwei Substanzen umfasst, die durch eine Trennwand gemäß Anspruch 12 voneinander abgetrennt sind, **dadurch gekennzeichnet, dass** es Folgendes umfasst:
- Spannen der obengenannten verformbaren Membran der Trennwand auf einem Träger,
- Füllen des ersten Fachs mit einer ersten Substanz über die Füllöffnung.
- Positionieren des besagten Trägers mit der gespannten Membran auf den Konturen eines internen Ansatzes des Fachs über die obengenannte Füllöffnung,
- Füllen des zweiten Fachs, das sich auf der anderen Seite der Membran des Fachs befindet, mit einer zweiten Substanz über die besagte Füllöffnung,
- und Schließen der besagten Füllöffnung des Fachs.

18. Verfahren zum Zusammenbau eines Fachs, das zwei einander gegenüberliegende Füllöffnungen mit mindestens zwei Substanzen umfasst, die
durch eine Trennwand gemäß Anspruch 12 voneinander abgetrennt sind, **dadurch gekennzeichnet, dass** es Folgendes umfasst:
- Spannen der obengenannten verformbaren Membran der Trennwand auf einem Träger,
- Positionieren des besagten Trägers mit der auf den Konturen eines internen Ansatzes des Fachs gespannten Membran,
- Füllen des ersten Fachs durch eine erste Substanz über die erste Füllöffnung,
- Füllen des zweiten Fachs, das sich auf der anderen Seite der Membran des Fachs befindet, mit einer zweiten Substanz über die zweite Füllöffnung,
- und Schließen der beiden Füllöffnungen der beiden Fächer des besagten Fachs.

## Claims

1. Partition for packaging creating at least two compartments firstly intended to cover the opening of at least one compartment in order to retain a substance placed inside so as to conserve the substance until usage, and secondly intended to be torn off in order to reveal said opening and let the substance out of said compartment and into the second one in order to be used, **characterised in that** the aforementioned partition is constituted of a malleable membrane which, stretched over the perimeter of the opening of said compartment in order to cover the latter, is susceptible, through bursting due to piercing or tearing, to retract over the perimeter of said opening and reveal the entire aforementioned opening, just as the membrane of a balloon which, when inflated with a gaseous fluid increases in volume and accepts flexion when pressed by hand whereas it bursts when put in contact with a rose thorn.

2. Partition set forth in claim 1, **characterised in that** the aforementioned malleable membrane of the partition is placed in a seal-proof manner over the perimeter of the opening of one of said compartments.

3. Partition set forth in claim 1, **characterised in that** the aforementioned malleable membrane of the partition is made in a flexible material.

4. Partition set forth in claim 1, **characterised in that** the aforementioned malleable membrane of the partition is made in an elastic and/or plastic material.

5. Partition set forth in claim 1, **characterised in that** the aforementioned malleable membrane of the partition is made in a basic material.

6. Partition set forth in claim 1, **characterised in that** the aforementioned malleable membrane of the partition is made in a composite material.

7. Partition set forth in claim 1, **characterised in that** the aforementioned malleable membrane of the partition is made in a single or multiple lamellar material.

8. Partition set forth in claim 1, **characterised in that** the aforementioned malleable membrane of the partition is made in a material comprising an insulating substance.

9. Partition set forth in claim 1, **characterised in that** the aforementioned malleable membrane of the partition is made in a material which is selective to gases.

10. Partition set forth in claim 1, **characterised in that** the aforementioned malleable membrane of the partition is placed directly over the perimeter of the opening of one of said compartments.

11. Partition set forth in claim 1, **characterised in that** the aforementioned malleable membrane of the partition is stretched over a support fitted to the perimeter of the opening of one of said compartments.

12. Partition set forth in any one of claims 1 to 11, **characterised in that** the malleable membrane of the aforementioned partition is stretched between at least two volumes of substances in order to firstly create a physical barrier between the volumes of said substances delimited by said membrane in at least one compartment for isolated substance conservation purposes and, secondly to create, through tearing and bursting of the stretched membrane via a spike and/or sharp object or pinching via twisting of the latter, a means for associating said volumes in order to ensure the mixing of said two substances so as to use the latter.

13. Method for assembling at least two compartments each retaining a substance separated from each other by a partition set forth in claim 12, **characterised in that** it consists in:
- stretching the aforementioned malleable membrane of the partition over the perimeter of the opening of a first compartment;
- filling the first compartment with a first substance and sealing said second compartment;
- filling the second compartment with a second substance through a filling orifice; and
- assembling the two compartments via sealing, fastening or screwing of the first compartment to the second compartment by joining together the perimeters of the openings of the two compartments so that the two compartments are separated with their opening facing each other, by means of the sealing partition created by the aforementioned separation membrane.

14. Method for assembling at least two compartments each retaining a substance separated from each other by a partition set forth in claim 12, **characterised in that** it consists in:
- filling the first compartment with a first substance through a filling orifice;
- sealing the aforementioned filling orifice of the first compartment by stretching the aforementioned malleable membrane of the partition over the perimeter of the filling orifice;
- filling the second compartment with a second substance through a filling orifice; and
- assembling the two compartments via sealing, fastening or screwing of the first compartment to the second compartment by joining together the perimeters of the two filling orifices of the two compartments so that the two compartments are separated with their opening facing each other, by means of the partition created by the aforementioned sealing membrane.

15. Method for assembling at least two compartments each retaining a substance separated from each other by a partition set forth in claim 12, **characterised in that** it consists in:
- stretching the aforementioned malleable membrane of the partition over the perimeter of the opening of a first compartment;
- filling the second compartment with a second substance through a filling orifice;
- assembling the two compartments via inserting in a seal-proof manner the first compartment in the intake opening of the second compartment by previously placing the membrane inside the second compartment so that the two compartments are only separated by the aforementioned sealing membrane;
- filling the first compartment with a first substance; and
- sealing the two compartments;

16. Method for assembling at least two compartments each retaining a substance separated from each other by a partition set forth in claim 12, **characterised in that** it consists in:
- filling the first compartment with a first substance through a filling orifice;
- sealing the aforementioned filling orifice of the first compartment by stretching the aforementioned malleable membrane of the partition over the perimeter of the orifice;
- filling the second compartment with a second substance through a filling orifice; and
- assembling the two compartments via inserting in a seal-proof manner the first compartment in the filling orifice of the second compartment by previously placing the membrane inside the second compartment so that the two compartments are only separated by the aforementioned sealing membrane.

17. Method for assembling a compartment comprising a single filling orifice of at least two substances separated from each other by a partition set forth in claim 12, **characterised in that** it consists in:
- stretching the aforementioned malleable membrane of the partition over a support;
- filling the first compartment of the compartment with a first substance through a filling orifice;
- positioning said support with the membrane stretched over the outline of an internal shoulder of the compartment, through the aforementioned filling orifice;
- filling the second compartment located on the other side of the membrane of the compartment with a second substance through said filling orifice; and
- sealing the aforementioned filling orifice of the compartment.

18. Method for assembling a compartment comprising two filling orifices facing each other of at least two substances separated from each other by a partition set forth in claim 12, **characterised in that** it consists in:
- stretching the aforementioned malleable membrane of the partition over a support;
- positioning said support with the membrane stretched over the outline of an internal shoulder of the compartment;
- filling the first compartment of the compartment with a first substance through the first filling orifice;
- filling the second compartment located on the other side of the membrane of the compartment with a second substance through the second filling orifice; and
- sealing the two filling orifices of the two compartments of the aforementioned compartment.
